# NEW EUROPEAN PATENT SPECIFICATION

(11) **EP 3 316 834 B2**
(45) Date of publication and mention of the opposition decision: **30.04.2025**
(45) Mention of the grant of the patent: 13.10.2021
(21) Application number: 16738964.2
(22) Date of filing: 30.06.2016
(51) Int. Cl.: A61F 13/15, A61F 13/49, B32B 5/26, B32B 7/14, B32B 27/12, B32B 37/12, B32B 37/20, B32B 37/22

(54) **STRETCH LAMINATE WITH INCREMENTALLY STRETCHED OR SELFED LAYER, METHOD FOR MANUFACTURING, AND DISPOSABLE ABSORBENT ARTICLE INCLUDING THE SAME**
STRETCHLAMINAT MIT SCHRITTWEISE GESTRECKTER ODER GESELFTER SCHICHT, VERFAHREN ZUR HERSTELLUNG UND ABSORBIERENDER EINWEGARTIKEL DAMIT
STRATIFIÉ EXTENSIBLE AVEC COUCHE AUTONOME OU ÉTIRÉE PROGRESSIVEMENT, PROCÉDÉ DE FABRICATION ET ARTICLE ABSORBANT JETABLE LE COMPRENANT

(30) Priority: 30.06.2015 US 201514755090
(43) Date of publication of application: 09.05.2018
(73) Proprietor: The Procter & Gamble Company, Cincinnati, OH 45202 (US)
(72) Inventor: WADE, Sarah, Marie, Cincinnati, Ohio 45202 (US); PHILLIPS, LeAnn, Nichole, Cincinnati, Ohio 45202 (US); LAVON, Gary, Dean, Cincinnati, Ohio 45202 (US); KLINE, Mark, James, Cincinnati, Ohio 45202 (US)
(74) Representative: P&G Patent Germany
(86) International application number: PCT/US2016/040268
(87) International publication number: WO 2017/004309

(56) References cited:
- EP-A1- 1 065 046
- EP-A1- 2 487 287
- WO-A1-00/56522
- WO-A1-2013/090519
- JP-A- 2008 029 836
- JP-A- 2008 149 015
- US-A- 5 650 214
- US-A1- 2004 192 140
- US-A1- 2006 121 252
- US-A1- 2013 082 418
- US-A1- 2013 306 226
- US-B2- 6 964 720

## Description

### BACKGROUND OF THE INVENTION

In order to maintain or grow their market share, manufacturers of disposable absorbent articles such as disposable diapers and absorbent pants must continue to discover and develop improvements to materials, components and features that affect aspects such as containment, absorbency, comfort, fit and appearance. Absorbent pants are manufactured in smaller sizes to be used as, *e.g.,* pull-on diapers and toilet training pants for young children, and in larger sizes to be used as, e.g., undergarments for persons suffering from incontinence.

A particular type of absorbent pant design currently marketed is sometimes called the "balloon" pant. The balloon pant design usually includes a central absorbent chassis and an elastic belt. The elastic belt is usually relatively wide (in the longitudinal direction) and elastically stretchable in the lateral direction. It entirely encircles the wearer's waist, and thereby covers a relatively large amount of the wearer's skin, and also makes up a relatively large portion of the visible outside surfaces of the pant. The central chassis portion is typically joined to the inside of the belt in the front, wraps under the wearer's lower torso between the legs, and is joined to the inside of the belt in the rear. The belt is often formed of two layers of nonwoven web sandwiching one or more elastic members such as a plurality of laterally-oriented strands or strips of elastomeric material, or a section of elastomeric film or elastomeric nonwoven. It is common among such designs that, in manufacture, the elastic member(s) are sandwiched between the nonwoven web layers in a strained condition. Upon completion of manufacture and allowance of the belt with sandwiched elastic member(s) to assume a relaxed condition, the elastic member(s) contract laterally toward their unstrained lengths. This causes the nonwoven web layers to form gathers that take the form of visible ruffles in the belt. It is believed that some consumers find the ruffles attractive because they present a textured, plush, frilly and/or soft appearance and feel, and also provide a visible indication of stretchability and comfortableness.
US 2004/0192140 discloses an elastomeric nonwoven laminate and a process for producing the elastomeric nonwoven laminate. The elastomeric nonwoven laminate includes a first nonwoven, a second nonwoven and a plurality of elastic strands. The plurality of elastic strands is attached to the first nonwoven while under a strain producing a corrugated intermediate laminate once the strain is relieved. A second nonwoven is joined to the corrugated intermediate laminate and subsequently mechanically activated forming an elastomeric nonwoven laminate.

The balloon pant design provides for certain efficiencies in manufacture, and it is believed that the design may gain popularity. Consequently, any improvement in components such as the belt that enhance its appearance and functionality may give the manufacturer thereof an advantage.

### BRIEF DESCRIPTION OF THE DRAWINGS

Fig. 1A is a perspective view of one example of a balloon pant.
Fig. 1B is a perspective view of another example of a balloon pant.
Fig. 2 is a schematic plan view of a balloon pant precursor structure, prior to joining of the front and rear sections of the belt.
Fig. 3 is a schematic, exploded perspective view of components of a belt.
Fig. 4 is a schematic, close-up plan view of a portion of a belt.
Fig. 5 is a schematic cross section of the portion of the belt shown in Fig. 4.
Fig. 6 is a schematic side view of an apparatus and process for incrementally stretching or SELFing a web material.
Fig. 7 is a schematic perspective view of a pair of stretching rollers.
Fig. 8 is a schematic cross sectional view of a portion of a web material as it passes through a nip between a pair of stretching members with intermeshing features.
Fig. 9 is a schematic plan view of portion of a web material after incremental stretching.
Fig. 10 is a schematic cross section of the portion of web material of Fig. 9.
Fig. 11 is a schematic perspective view of a pair of stretching rollers.
Fig. 12 is a schematic plan view of a portion of a web material after SELFing.
Fig. 13 is a schematic perspective view of a portion of a web material after SELFing.
Fig. 14 is a schematic perspective view of a pair of stretching rollers.
Fig. 15 is a schematic perspective view of surfaces of a pair of stretching members with intermeshing features.
Fig. 16 is a schematic plan view of a portion of a web material after SELFing.
Fig. 17 is a schematic plan view of a portion of a web material after SELFing.
Fig. 18 is a schematic, close-up plan view of a portion of a belt having a SELFed layer.
Fig. 19 is a schematic cross-direction view of one alternative arrangement of components and a process used in a method for manufacturing a stretch laminate material as described herein.

### DETAILED DESCRIPTION OF THE INVENTION

"Cross direction" (CD) - with respect to the making of a nonwoven web material, the nonwoven material itself, a laminate thereof, or an article in which the material is a component, refers to the direction along the material substantially perpendicular to the direction of forward travel of the material through the manufacturing line in which the material and/or article is manufactured.

Throughout the present description, a material or composite of materials is considered to be "elastic" or "elastomeric" if, when a biasing force is applied to the material, the material or composite can be extended to an elongated length of at least 150% of its original relaxed length *(i.e.* can extend at least 50%), without rupture or breakage which substantially damages the material or composite, and when the force is removed from the material or composite, the material or composite recovers at least 40% of such elongation. In various examples, when the force is removed from an elastically extensible material, the material or composite may recover at least 60% or even at least 80% of its elongation.

"Film" means a skin-like or membrane-like layer of material formed of one or more polymers, which does not have a form consisting predominately of a web-like structure of consolidated polymer fibers and/or other fibers.

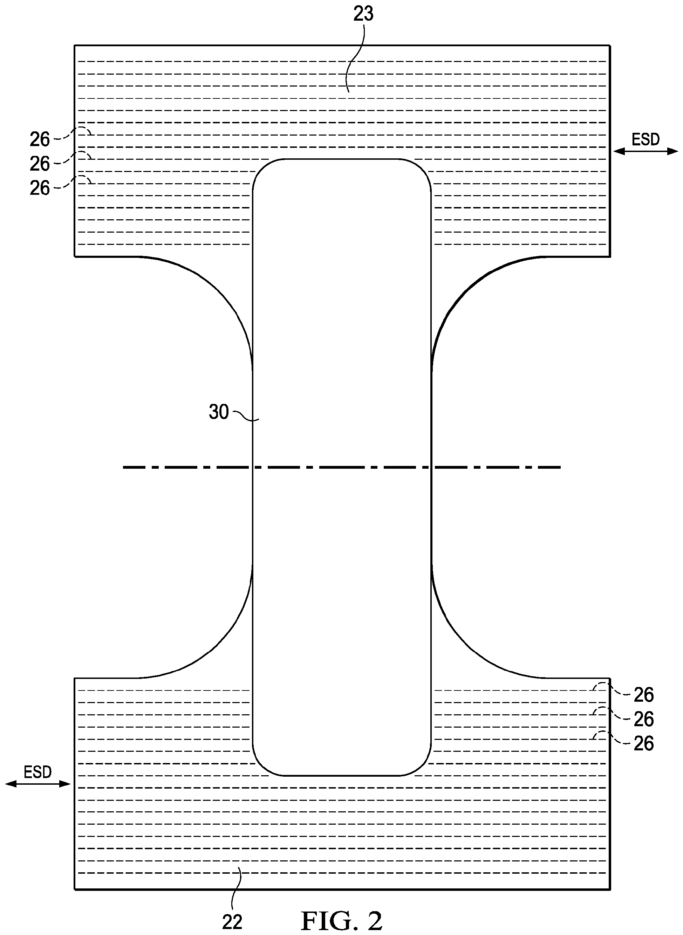

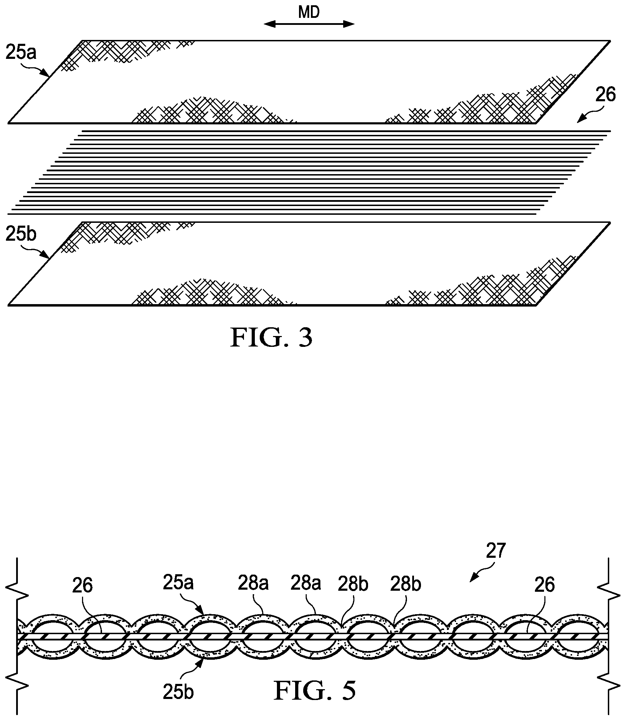

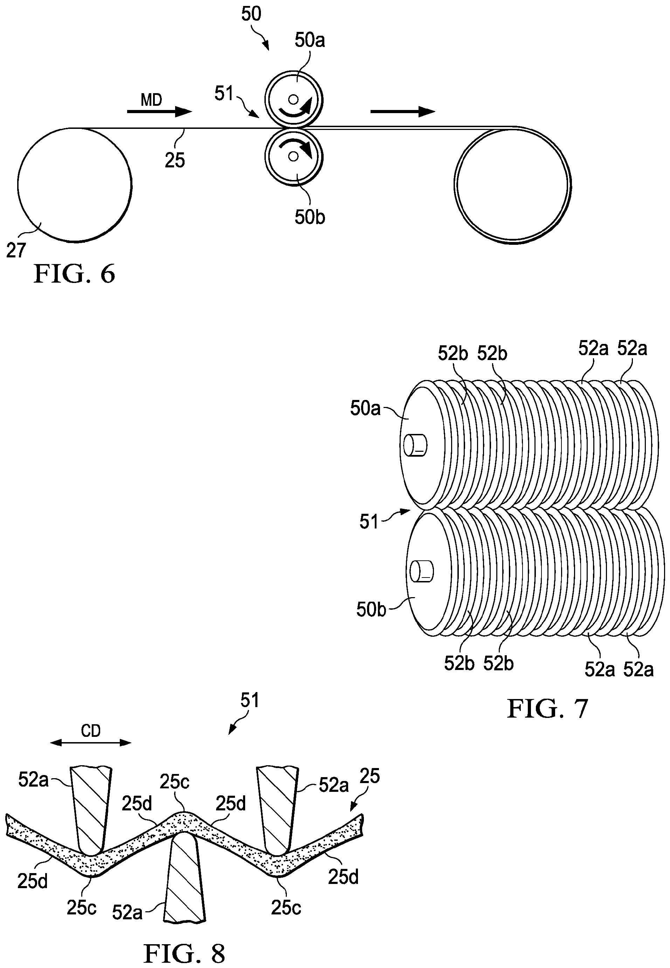

"Incremental stretching" (and forms thereof) refers to the process in which a web material is controllably plastically stretched in increments along one or more directions by being passed under tension between the surfaces of a pair of stretching members having continuously intermeshing teeth, ridges and valleys or other features, such as described in, for example, U.S. Pat. App. Pub. Nos. US 2013/0082418 (and by way of particular example but without limitation, features depicted in Figs. 5B, 7A and 7B thereof) and US 2002/0105110 (and by way of particular example but without limitation, features depicted in Fig. 2 thereof). The stretching members may be a pair of rollers (*e.g*., "ring" rollers), gear-like rollers, belts or plates with intermeshing features. For purposes herein, "incremental stretching" is distinguished from "SELFing" in that the intermeshing features of the stretching members are continuously intermeshing such that no substantial portion of the web material passing between the surfaces is left unaffected.

"Lateral" - with respect to a pant and its wearer, refers to the direction generally perpendicular with the wearer's standing height, or the horizontal direction when the wearer is standing.

"Longitudinal" - with respect to a pant and its wearer, refers to the direction generally parallel with the wearer's standing height, or the vertical direction when the wearer is standing. "Longitudinal" is also the direction generally parallel to a line extending from the midpoint of the front waist edge to the midpoint of the rear waist edge.

"Machine direction" (MD) - with respect to the making of a nonwoven web material, the nonwoven material itself, or a laminate thereof, refers to the direction along the material or laminate substantially parallel to the direction of forward travel of the material or laminate through the manufacturing line in which the material or laminate is manufactured.

"Machine direction bias," with respect to the fibers forming a nonwoven web, means that a majority of the fibers, as situated in the web (prior to any incremental stretching or SELFing of the web), have lengths with machine direction vector components that are greater than their cross direction vector components.

A "nonwoven" is a manufactured sheet or web of directionally or randomly oriented fibers which are first formed into a batt and then consolidated and bonded together by friction, cohesion, adhesion or one or more patterns of bonds and bond impressions created through localized compression and/or application of pressure, heat, ultrasonic or heating energy, or a combination thereof. The term does not include fabrics which are woven, knitted, or stitchbonded with yarns or filaments. The fibers may be of natural and/or man-made origin and may be staple and/or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes including but not limited to meltblowing, spunbonding, spunmelting, solvent spinning, electrospinning, carding, film fibrillation, melt-film fibrillation, airlaying, dry-laying, wetlaying with staple fibers, and combinations of these processes as known in the art. The basis weight of nonwoven fabrics is usually expressed in grams per square meter (gsm).

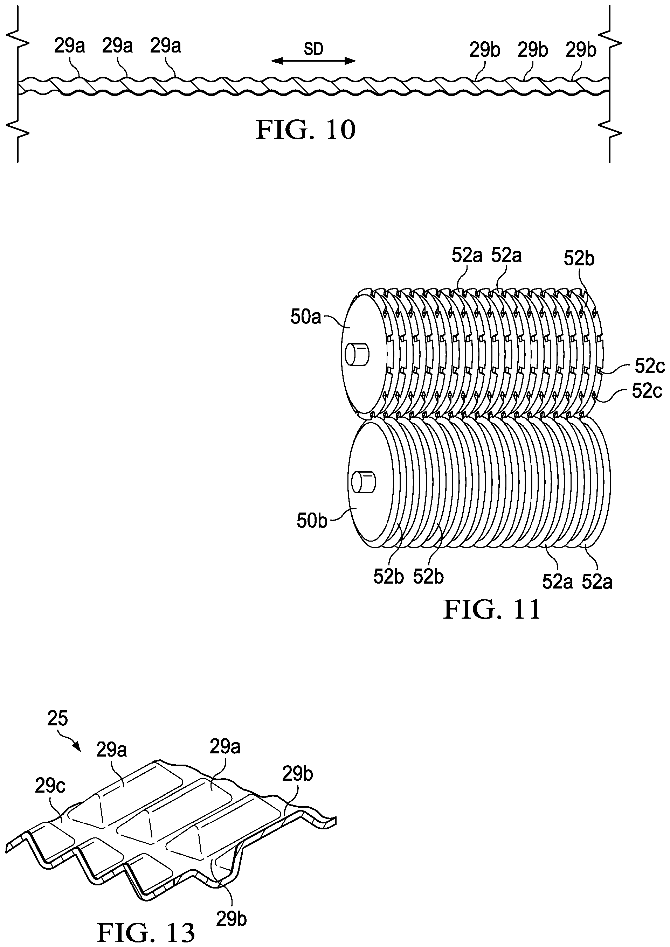

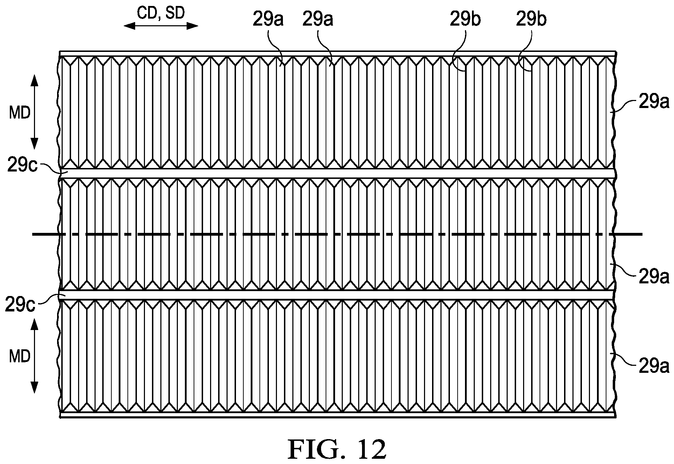

"SELFing" (and forms thereof) refers to a process in which a web material is controllably plastically stretched in increments along one or more directions by being passed under tension between the surfaces of a pair of stretching members having discontinuously intermeshing teeth, ridges and valleys or other features, such as described in, for example, U.S. Pat. No. 5,650,214; and U.S. Pat. App. Pub. Nos. US 2013/0082418 (and by way of particular example but without limitation, features depicted in Fig. 11 thereof) and US 2002/0105110 (and by way of particular example but without limitation, features depicted in Fig. 6 thereof). The stretching members may be a pair of rollers (*e.g.*, "ring" rollers), gear-like members, belts or plates with intermeshing features. For purposes herein, "SELFing" is distinguished from "incremental stretching" in that the intermeshing features of the stretching members are discontinuously intermeshing such that portions of the web material (for example, bands, or patterns thereof) are left with relatively lower or no deformation. Non-limiting examples of "SELFed" material are depicted in U.S. Pat. App. Pub. Nos. US 2013/0082418, Figs. 12-14, showing areas 72 in which relatively lower or no plastic deformation has occurred, forming patterns of bands of relatively unstretched material. For purposes herein, "SELFing" is not limited to a process to which a film web is subjected, but includes processes to which a nonwoven web may be subjected.

"z-direction," with respect to a web, means generally orthogonal or perpendicular to the plane approximated by the web along the machine and cross direction dimensions.

Although examples of the structure of the invention are described herein as used to form the belt of a balloon-type absorbent pant, it will be appreciated that examples may be used to form other components of pants, diapers, other wearable articles, and other products as well.

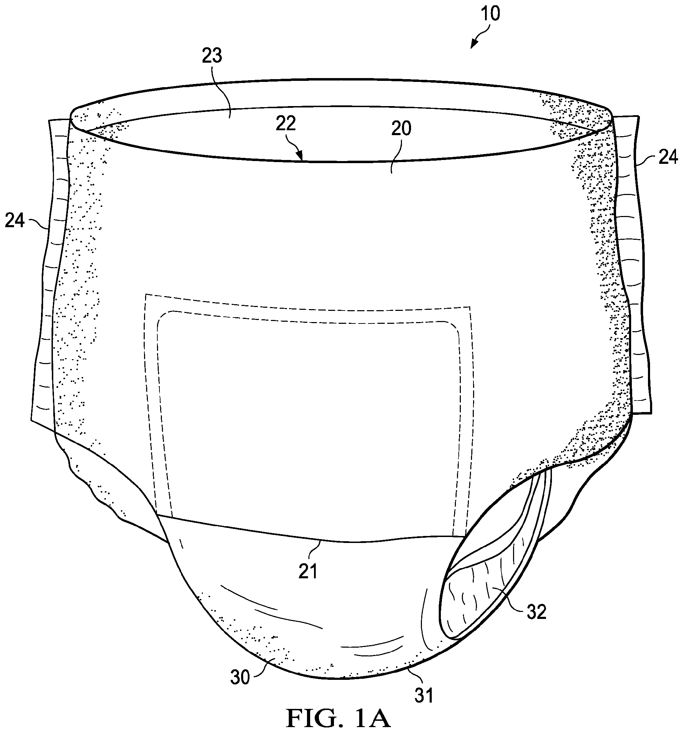

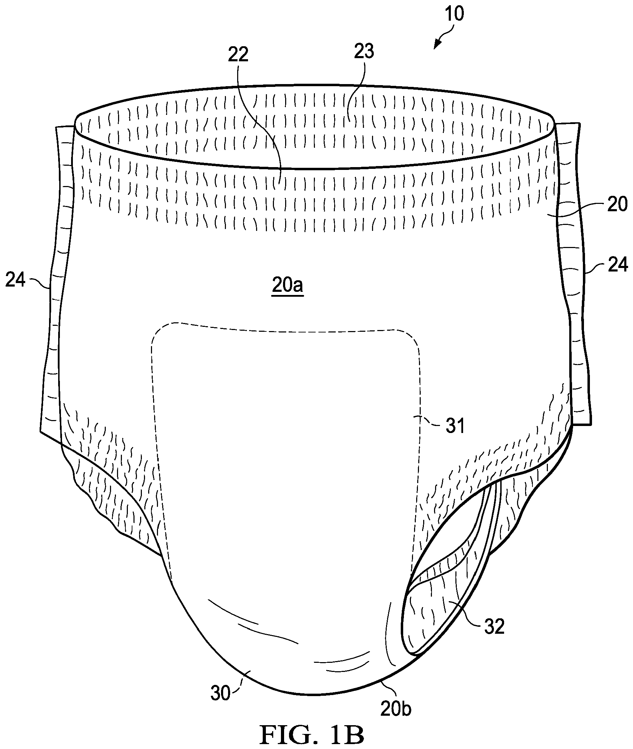

Figs. 1A and 1B depict examples of balloon-type absorbent pants 10. The pant may include a panel structure in the form of a belt 20 supporting a central chassis 30. Central chassis 30 may include any combination of components found in disposable diapers and absorbent pants, including but not limited to a liquid impermeable backsheet 31, a liquid permeable topsheet (not shown), an absorbent core structure (not shown), and elasticized barrier cuffs 32. Examples and descriptions of components and configurations of a central chassis may be found in US2013/0211355, wherein the chassis described includes components and features that may be included in central chassis 30.

In the example shown in Fig. 1A, belt 20 stops short of the crotch region 12 of the pant, at lower edge 21. In the example shown in Fig. 1B, belt 20 is part of an outer structure that includes a belt portion 20a encircling the wearer's waist, an outer wrap portion 20b that overlies the central chassis to the outside thereof and wraps thereabout through the crotch region. The outer wrap portion 20b may be formed of a layer of nonwoven web, which also serves as the outer layer of the belt portion 20a. The belt may have front and rear portions 22, 23, which are joined together at seams 24, and define, respectively, front and rear waist regions of the pant.

Fig. 2 schematically depicts a structure that is the precursor to a pant such as depicted in Fig. 1B, prior to joining of front and rear portions 22, 23 at seams 24 as depicted in Figs. 1A and 1B. Central chassis 30 overlies front and rear portions 22, 23 to the inside thereof. Front and rear portions of the belt 22, 34 each have an elastic stretch direction ESD along which the belt may elastically stretch laterally relative a wearer, which accommodates donning of the pant and provides for snug, comfortable and neat fit on the wearer.

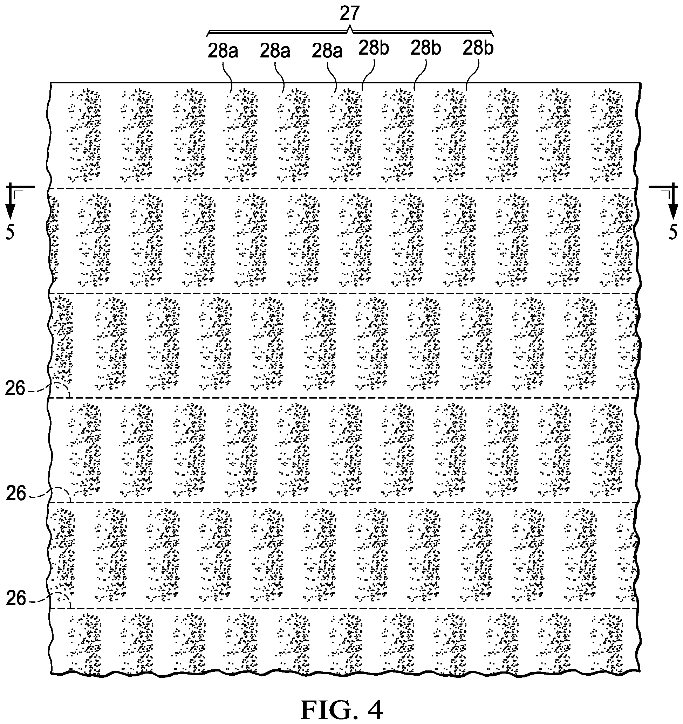

Referring to Figs. 3-5, belt 20 may be formed of layers of nonwoven web 25a, 25b, which respectively form inner and outer layers of the belt. Suitable nonwoven web materials that may be useful in the present invention also include, but are not limited to spunbond, spunlaid, meltblown, spunmelt, solvent-spun, electrospun, carded, film fibrillated, melt-film fibrillated, air-laid, dry-laid, wet-laid staple fibers, and other and other nonwoven web materials formed in part or in whole of polymer fibers, as known in the art. The nonwoven web may be formed predominately of polymeric fibers. In some examples, suitable non-woven fiber materials may include, but are not limited to polymeric materials such as polyolefins, polyesters, polyamide, or specifically, polypropylene (PP), polyethylene (PE), poly-lactic acid (PLA), polyethylene terephthalate (PET) and/or blends thereof. In some examples, the fibers may be formed of PP/PE blends such as described in U.S. Pat. No. 5,266,392. Nonwoven fibers may be formed of, or may include as additives or modifiers, components such as aliphatic polyesters, thermoplastic polysaccharides, or other biopolymers. Further useful nonwovens, fiber compositions, formations of fibers and nonwovens and related methods are described in U.S. Pat. No. 6,645,569; U.S. Pat. No. 6,863,933; and U.S. Pat. No. 7,112,621; and in co-pending US20030148684, and US20050008839 and US9636890.

The individual fibers may be monocomponent or multicomponent. The multicomponent fibers may be bicomponent, such as in a core-and-sheath or side-by-side arrangement. Often, the individual components comprise polyolefins such as polypropylene or polyethylene, or their copolymers, polyesters, thermoplastic polysaccharides or other biopolymers.

According to one example, the nonwoven may comprise a material that provides good recovery when external pressure is applied and removed. Further, according to one example, the nonwoven may comprise a blend of different fibers selected, for example from the types of polymeric fibers described above. In some embodiments, at least a portion of the fibers may exhibit a spiral curl which has a helical shape. According to one example, the fibers may include bicomponent fibers, which are individual fibers each comprising different materials, usually a first and a second polymeric material. It is believed that the use of side-by-side bi-component fibers is beneficial for imparting a spiral curl to the fibers.

In order to enhance softness perceptions of the laminate, nonwovens may be treated by hydrojet impingement, which may also be known as hydroenhancement, hydroentanglement or hydroengorgement. Such nonwovens and processes are described in, for example, U.S. Pats. Nos. 6,632,385 and 6,803,103, and U.S. Pat. App. Pub. No. 2006/0057921.

Other examples of nonwoven web that may be useful in the present laminate may be an SMS web (spunbond-meltblown-spunbond web) made by Avgol Nonwovens LTD, Tel Aviv, Israel, under the designation XL-S70-26; an SSS (spunbond-spunbond-spunbond) web made by Pegas Nonwovens AS in Znojmo, Czech Republic, under the designation 18 XX 01 00 01 00 (where XX = the variable basis weight); an SSS web made by Gulsan Sentetik Dok San VE TIC AS, in Gaziantep, Turkey, under the designation SBXXF0YYY (where XX = the variable basis weight, and YYY = the variable cross direction width); an HESB (hydroenhanced spunbond) web made by First Quality Nonwovens Inc., in Hazelton, Pennsylvania, under the designation SEH2503XXX (where XXX = the variable cross direction width); and a bicomponent SS web.

A nonwoven web useful as a component to form one or both of layers 25a, 25b may be pre-bonded, prior to aperturing as described below. A batt of fibers may be calendered and pre-bonded in a pattern, to consolidate the batt/fibers and create a pattern of bonds that adds tensile strength and dimensional stability, converting the batt of fibers to a coherent and useable nonwoven web material. The web may be imparted with a pattern of pre-bonding as described in, for example, U.S. Pat. No. 5,916,661 (pre-bonding in a pattern of "point calendered bonds 200 to form a coherent web structure") and co-pending US2013/0324956 (pattern of "primary fiber bonds"). The pre-bonding may consist of a pattern of thermal bonds, mechanical bonds or adhesive bonds, although in some circumstances thermal bonding may be preferred.

Layers of nonwoven web 25a, 25b may sandwich one or more elastic members such as a plurality of strands 26 of an elastomeric material, such as an elastane (for example, LYCRA HYFIT fiber, a product of Invista, Wichita, Kansas). Layers of nonwoven web 25a, 25b may be joined together about elastic strands 26 by adhesive deposited between the layers, by thermal bonds, by compression bonds, or by a combination thereof. In other examples, the one or more elastic members may be strips or a section of film formed of elastomeric material.

The elastomeric members can also be formed from various other materials, such as but not limited to, rubbers, styrene ethylbutylene styrene, styrene ethylene propylene styrene, styrene ethylene ethylene propylene styrene, styrene butadiene styrene, styrene isoprene styrene, polyolefin elastomers, elastomeric polyurethanes, and other elastomeric materials known in the art, and combinations thereof. In some embodiments, the elastic members can be extruded strand elastics with any number of strands (or filaments). The elastomeric members can have a decitex ranging from 50 to 2000, or any integer value for any decitex value in this range, or any range formed by any of these integer values. The elastomeric members may be in a form of film. Examples of films have been described extensively in prior patent applications *(see,* for example, U.S. Pat. App. Pub. No. 2010/0040826). The film may be created with a variety of resins combined in at least one of several sublayers, the latter providing different benefits to the film.

During manufacture of the belt structure, the elastic member such as elastic strands 26 may be strained lengthwise by a desired amount as they are being incorporated into the belt structure. Upon subsequent relaxation of the belt, the elastic member such as elastic strands 26 will contract toward their unstrained lengths. This causes the layers of nonwoven material 25a, 25b to gather and form ruffles 27 having peaks 28a and depressions 28b generally transverse to the lengths of the elastic strands 26.

It may be appreciated that the size(s) and shape(s) of the ruffles 27 will be affected, and may be manipulated, by design of the pattern of joined portions and/or bonding between the layers of nonwoven web 25a, 25b, with respect to each other and with respect to elastic strands 26. When joining and/or bonding are effected using adhesive deposited upon one or both layers 25a, 25b prior to lamination, the adhesive may be deposited in a pattern. Examples of methods for applying patterned deposits of adhesive to a nonwoven web substrate to enable manufacture of an elasticized laminate are described in U.S. Pat. No. 8,186,296. The pattern selected may be effected by design of a correspondingly designed roller. The pattern of adhesive to be applied may be designed to affect the size(s) and shape(s) of the ruffles 27. The layers 25a, 25b may be adhesively joined and/or bonded to each other at the locations of adhesive deposits, and remain unjoined or unbonded, or free, of each other at other locations, such that they may move and shift slightly relative each other as the laminate is moved and stretched, as during wear of the article. Similarly, when joining and/or bonding is effected using thermal calender bonding, the joining and/or bonding pattern may be designed to affect the size(s) and shapes of the ruffles 27. In one example, deposits of adhesive bonding layers 25a, 52b together may be included along lines substantially parallel the elastic strands 26. In another example, deposits of adhesive bonding layers 25a, 52b together may be included along lines substantially perpendicular the elastic strands 26.

The ruffles 27 impart some z-direction loft and texture to the belt. It has been found, however, that z-direction loft and texture of the belt may be further enhanced in surprising and esthetically pleasing ways by additional processing of one or both of the layers 25a, 25b prior to formation of the belt.

Incremental stretching (sometimes known as "activation") techniques have heretofore been used to impart elastic extensibility to a laminate of, *e.g.,* one or more layers of nonwoven web material and an elastic film layer or other arrangement of sandwiched elastic member(s). The nonwoven web layer(s) may be relatively inelastic. Thus, following lamination of the nonwoven web layer(s) with the elastic member(s), the laminate may be relatively inelastic despite the relative elasticity of the elastic member(s). When the laminate is incrementally stretched, or "activated," fibers of the nonwoven layer(s) may be incrementally plastically stretched or even broken to some extent, which allows the nonwoven layer(s), though still adhered to the elastic member(s), to extend and accommodate stretch with the elastic member(s). Incremental stretching may be directional, such that the nonwoven material is plastically stretched or broken along one (*e.g.,* the stretch) direction but not the direction perpendicular thereto.

SELFing techniques have heretofore been used to impart enhanced elastic-like qualities to polymer films such as polyethylene films that have relatively low elasticity. *See, e.g.,* U.S. Pat. No. 5,650,214. When the film is SELFed, discrete, discontinuous areas of the film are plastically stretched, while other continuous areas are left relatively unstretched. The stretched and unstretched areas are arranged in a pattern, with the unstretched areas forming bands of relatively unstretched film, or a network of such bands along the film. The SELFed film may then stretch somewhat under tensile force along the direction of the stretching of the stretched areas, while the unstretched areas resist stretching and tend to cause the film to retract when the tensile force is removed, imparting enhanced elastic-like behavior to the film. An example of SELFed film appears in consumer products in GLAD FORCEFLEX brand trash bags, a product of The Glad Products Company, Oakland, CA.

A single nonwoven web layer instead of a laminate may be incrementally stretched. Similarly, a nonwoven material instead of a film material may be SELFed. It has been found that when either process is applied to a nonwoven layer used to make the belt as described herein, surprising and esthetically pleasing effects can result. For example, an incrementally stretched or SELFed nonwoven web layer component of a belt may impart a pleasing textured or even terry cloth-like appearance to the belt, and also add z-direction loft, as compared with a belt formed of nonwoven web layers not so processed.

Referring to Figs. 6-8, a nonwoven web 25 may be passed or conveyed from an upstream supply 27 between the mating surfaces of a pair of stretching members 50. In the example depicted in Fig. 6, the pair of stretching members 50 are a pair of stretching rollers 50a, 50b proximately arranged with rotational axes in parallel along the cross direction to form a nip 51 therebetween. Rollers 50a, 50b each may have formed thereon a series of circumferential ridges 52a and valleys 52b that rotate in planes parallel to the machine direction MD. The ridges 52a and valleys 52b of respective stretching rollers 50a, 50b, and the rollers, may be configured and arranged to intermesh without points of contact between them that may form pinch points at which squashing or severing of the web fibers might occur. Referring to Fig. 8, as nonwoven web 25 passes through nip 51, it is stretched in increments in the cross direction, at stretch zones 25d lying between contact zones 25c which contact the ridges. The extent of the increments of stretch corresponds to the distance between the adjacent ridges 52a on the respective stretch rollers. In stretch zones 25d, the fibers of the nonwoven web may be plastically stretched, and some may even break, depending upon the composition and ductility of the fibers, the directional bias of the fibers (or lack thereof), the radial height of ridges 52a and depth of valleys 52b, the depth of intermeshing of the ridges/valleys of the respectively stretching rollers 50a, 50b, and the machine direction speed of the nonwoven web 25 as it passes through nip 51.

The configuration and process schematically depicted in Figs. 6-8 is sometimes called "ring rolling." In other alternatives, the pair of stretching members may be a belt and roller or pulley with intermeshing features, or pair of belts with intermeshing features, arranged to intermesh such as depicted and described in, for example, U.S. Pat. App. Pub. No. US 2013/0082418.

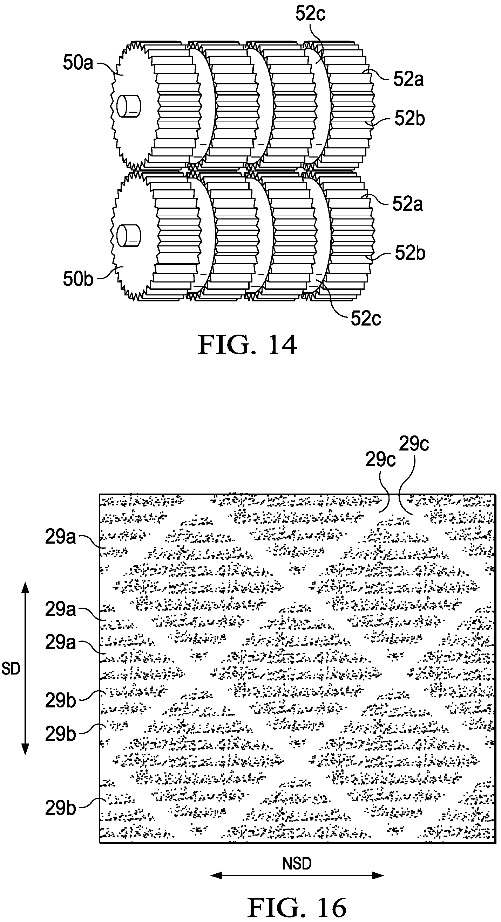

In still another alternative using rollers, the ridges and valleys on stretching rollers 50a, 50b, rather than being circumferential about the stretching rollers, may be longitudinal, *i.e.,* parallel with the rotational axes of the rollers and extending along the cross direction, with configurations resembling, and intermeshed in the manner of, a pair of engaged spur gears. An example of the latter configuration is suggested in Fig. 14. In yet another alternative (not shown), the stretching rollers 50a, 50b may be configured and arranged to intermesh in the manner of a pair of engaged helical gears.

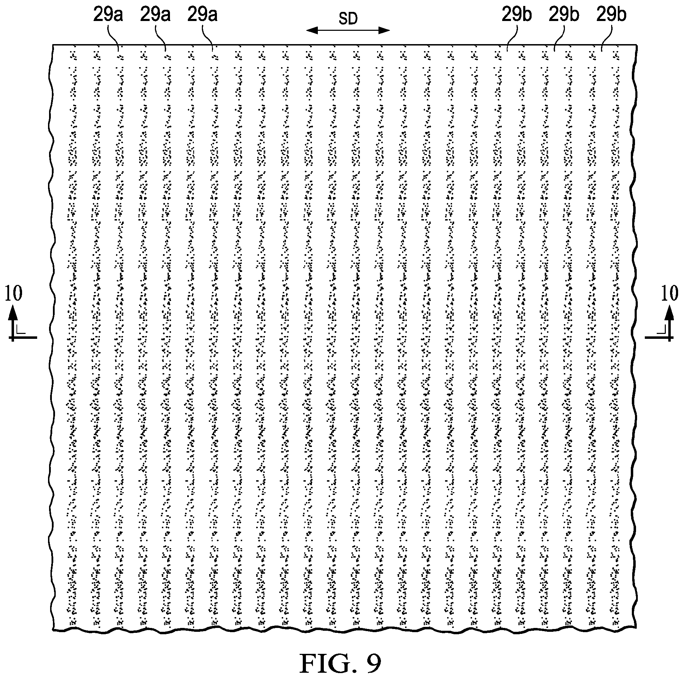

Incrementally stretching a nonwoven web material in the manner described above will result in a nonwoven web material having an appearance schematically depicted in Figs. 9 and 10. The resulting web will have a series of generally linear ridges 29a and valleys 29b, corresponding with the ridges and valleys of the stretching members, that impart an appearance to the nonwoven web resembling corduroy material. The material has been incrementally stretched along a direction (stretched direction SD) generally perpendicular to the directional orientation of the ridges 29a and valleys 29b. It will be appreciated that stretched direction SD will be perpendicular to the machine direction in, for example, a ring rolling operation, and parallel to the machine direction when the stretching rollers are configured like, for example, intermeshing spur gears.

As an alternative to incremental stretching as described above, a nonwoven web 25 used to form a layer of a belt 20 may be SELFed prior to formation of the belt. For example, referring to Fig. 11, a pair of ring-rolling type stretching rollers 50a, 50b may be configured and arranged to stretch a nonwoven web in a manner similar to that described above. In an example of a SELFing process, one of the stretching rollers such as roller 50a, may be configured with breaks or interruptions 52c in the circumferential ridges 52a. As suggested in Fig. 11, the breaks or interruptions 52c may be regularly arranged along rows parallel to the machine direction. In another alternative, however, they may be arranged helically or in any other configuration desired. The breaks or interruptions 52c may be configured to leave portions of the nonwoven web relatively less stretched, or unstretched, in a desired pattern.

Appropriately configured, a pair of stretching rollers 50a, 50b can cause the nonwoven web to be SELFed in a pattern resembling that depicted in Figs. 12 and 13. In that example, the nonwoven web 25 bears a pattern of ridges 29a and valleys 29b that are discontinuous, interrupted by bands 29c of material that is relatively less stretched, or unstretched. The bands shown in Fig. 12 reflect the pattern of interruptions 52c on the stretching roller 50a (Fig. 11) and are configured as regularly-spaced, parallel bands lying along the cross direction CD, which coincides with the stretched direction. It will be appreciated that the configuration of these bands may be changed as desired by changing the pattern of interruptions 52c on a stretching roller or other stretching member. Another example of a pair of stretching rollers that may be chosen to create a pattern of bands as shown in Figs. 12 and 13 is shown in Fig. 14. In the stretching roller configuration of Fig. 14, the bands 29c of the resulting SELFed material would lie along the machine direction rather than along the cross direction, and the stretch direction would also lie along the machine direction. It will be appreciated that a configuration of stretching rollers like that reflected in Fig. 11, or alternatively, a configuration like that reflected in Fig. 14, may be employed according to the orientation of the stretch direction and of the bands 29c desired in the SELFed nonwoven web material.

When a web material is SELFed, the relatively less stretched or unstretched bands 29C cause the material to more effectively or more closely retain its pre-stretched dimension along the longitudinal orientation of the bands 29c, when the material is not under tension, as compared with a material that is incrementally stretched. The bands 29c may also cause the material to retain more of its tensile strength along the direction of the bands.

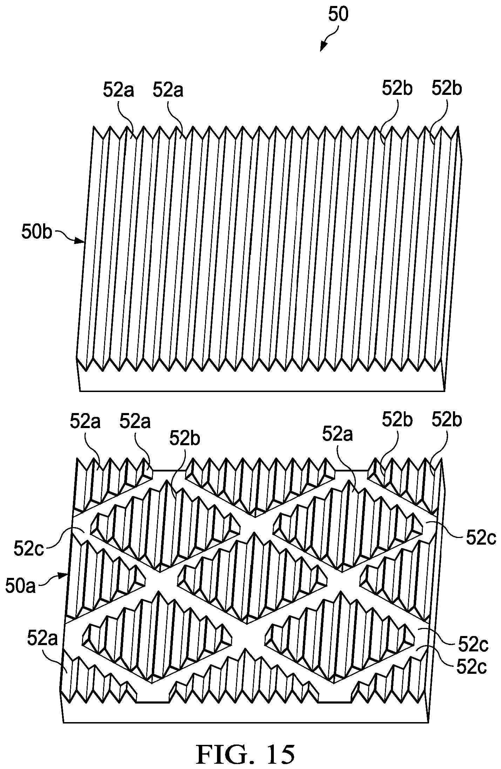

From the foregoing it will be appreciated that stretching members 50 may be configured to impart differing patterns of stretched areas and relatively less stretched bands to a web material in a SELFing process. Referring to Fig. 15, a pair of stretching members may include first stretching member 50a and second stretching member 50b. Stretching members 50a and 50b may be formed with patterns of ridges 52a and valleys 52b, each stretching member's ridges and valleys configured to intermesh with those of the other. For purposes of a SELFing process, one of the stretching members such as stretching member 50a may further include a pattern of interruptions 52c in the ridges 52a. As reflected in Fig. 15, the pattern of interruptions may be configured as a regular pattern of crossing pathways. Desired configurations of intermeshing features of stretching members 50a, 50b such as, for example, those suggested in Fig. 15, may be imparted to pair of intermeshing flat plates, to a pair of intermeshing rollers, belts, etc. forming a pair of stretching members.

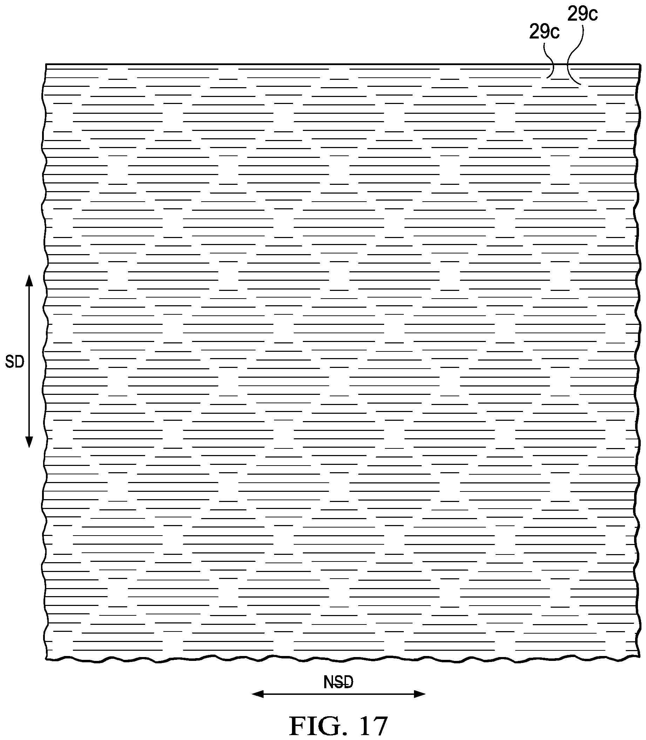

When a nonwoven web material is passed between an intermeshing pair of stretching members having the features in the example reflected in Fig. 15, the web material may be imparted with a pattern of stretched areas having ridges 29a and valleys 29b, defined by a pattern of bands 29c of relatively less stretched or unstretched material - as reflected in Figs. 16 and 17. The stretched direction SD is the direction perpendicular to the orientations of the ridges 29a and valleys 29b. Generally, this means that the SELFed material will, subsequent to SELFing, stretch more easily in the stretched direction than in the non-stretched direction NSD perpendicular to it - but this may also be affected by the directional bias of the fibers in the nonwoven and the extent of stretching to which the material is subjected in the SELFing process. As noted the bands 29a of relatively less stretched or unstretched material help the SELFed material better retain its unstretched dimensions, as compared with an incrementally stretched material.

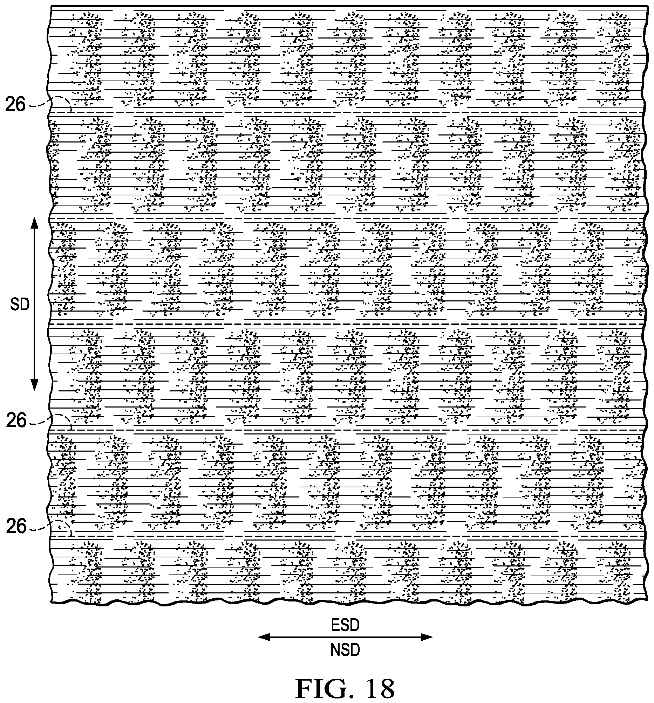

Referring now to Figs. 3-5, 9-10, 16, 17 and 18, one or both of the nonwoven web layers 25a, 25b forming a belt may be incrementally stretched or SELFed according to any of the examples described above, or any other alternatives thereto, prior to formation of the belt. As may be appreciated from Fig. 18, a nonwoven layer bearing a pattern of incremental stretching or SELFing may add surprising and esthetically pleasing visual complexity and texture to the belt and give it a more cloth-like appearance, *e.g*., an appearance of terrycloth. The pattern ridges 29a and valleys 29b from incremental stretching or SELFing may combine with the pattern of ruffles 27 with peaks 28a and depressions 28b resulting from formation of the belt with pre-strained elastic strands 26 to add such visual complexity and texture. Because such nonwoven web material will have its own enhanced z-direction loft *(see, e.g.,* Figs. 10 and 13), use of such material to form a belt layer will enhance the loft of the belt as well, as compared with a belt formed of material that is not incrementally stretched or SELFed.

When an incrementally stretched or SELFed nonwoven web material is used form one or both layers 25a, 25b of a belt, it may be desired that the stretch direction SD of the stretched web material be oriented in a direction not parallel with the elastic stretch direction ESD of the belt, more preferably forming an angle of 45 degrees or greater with elastic stretch direction ESD of the belt, and even more preferably approaching an angle of 90 degrees (substantially perpendicular) with elastic stretch direction of the belt, rather than approximately or substantially parallel thereto. This is because aligning the stretch direction SD of the web material substantially parallel to the elastic stretch direction ESD of the belt may result in permanent elongation of the web material along the elastic stretch direction when the belt is stretched therealong, which may result in giving the belt the appearance of becoming delaminated or otherwise falling apart when the belt contracts along the elastic stretch direction and the permanently elongated web is forced to gather therealong.

In some circumstances a SELFed nonwoven web rather than an incrementally stretched web may be preferred to be used as one or both of layers 25a, 25b of the belt. This may be desired for one or more of several reasons. As noted, the bands 29c of relatively less stretched, or unstretched, SELFed nonwoven web material help the material retain dimensional stability and reduce the chance that the material will permanently elongate and create a sloppy appearance when the pant including the belt is worn. Further, a pattern of adhesive bonding the nonwoven layers 25a, 25b together may be applied to the SELFed nonwoven web layer at the locations of, or corresponding with the bands 29c, thereby allowing for a sharper visual definition of the stretched areas of ridges and valleys 29a, 29b.

In a particular example, a belt may be constructed such that the fibers of a nonwoven web layer 25a (and/or 25b) have a machine direction bias substantially parallel with the elastic stretch direction ESD; and the layer may be incrementally stretched or SELFed with a stretch direction SD substantially perpendicular to the elastic stretch direction ESD. It is believed that this particular combination of features may provide a belt structure with the greatest tensile strength in the elastic stretch direction and structural integrity, while taking advantage of the texture effects of incremental stretching or SELFing.

The loft and visual complexity added by incremental stretching or SELFing may contribute to tactile and visual perceptions of added softness and/or breathability of the belt.

It may be appreciated that the pattern of SELFing selected may be coordinated with the pattern of adhesive selected to adhere the laminate, for varying effects. Referring to Fig. 18, for example, a pattern of SELFing may be selected that is somewhat independent of the pattern of ruffles created by a pattern of adhesive. The adhesive pattern may be selected so as to provide, for example, orderly machine direction rows but disordered or random cross direction columns of ruffles 27. The pattern of SELFing may be sized and ordered so as to fall randomly on the ruffles 27 in the machine and/or cross directions. As a result, the stretched area will be positioned relative the ruffles in a somewhat random fashion, providing a particular visual effect. In another example (not shown), the pattern of adhesive may be selected to provide substantially orderly machine direction rows and cross-direction columns of ruffles. The SELFing may be patterned, for example, so as to cause stretched areas to fall on the peaks 28a of the ruffles 27, in, for example, substantially evenly-spaced rows and substantially evenly-distributed numbers. In this latter example, the stretched areas are positioned substantially at the peaks 28a of the ruffles 27 at a location on the nonwoven web layer at which they will experience the most movement (having another visual effect), as the belt is stretched and moved, as during wear of the article. Similarly, the stretched areas may be patterned in coordination with the spacing between the elastic members such as strands 26, such that they are substantially evenly distributed relative the locations of the strands 26 in the belt.

Nonwoven web materials of the type typically used to form such belts are generally highly breathable. (Breathability, typically reflected in measurable vapor permeability of the material, is desired to avoid overhydration of the wearer's skin beneath the article.) Accordingly, it not necessary or desirable to provide incremental stretching or SELFing merely for the purpose of increasing breathability. Because the materials are already highly breathable incremental stretching or SELFing may have little effect in this regard. However, it is believed that the visible presence of patterns of stretched areas and loft in the material may in some circumstances give consumers the impression of high breathability, or reinforce or increase such impression - which may provide a marketing advantage for the manufacturer.

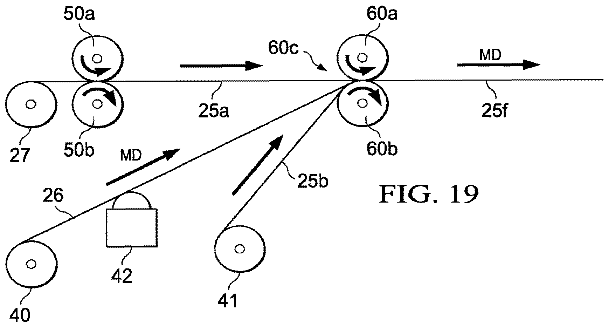

A method for forming a highly-textured stretch laminate may be described with reference to Fig. 19. A first nonwoven material layer 25a may be withdrawn from a supply thereof 27 and passed through the nip between a pair of incremental stretching rollers 50a, 50b with their axes of rotation oriented in a cross direction, thereby incrementally stretching layer 25a. In an alternative the rollers 50a, 50b may be SELFing rollers. Following incremental stretching or SELFing, layer 25a may be conveyed toward the nip 60c between a pair of laminating rollers 60a, 60b. A second nonwoven material layer 25b may also be withdrawn from a supply 41, and conveyed toward the laminating rollers. Second nonwoven material layer 25b is incrementally stretched or SELFed, in addition to first material layer 25a, prior to lamination. Elastic member(s) 26 may be withdrawn from a supply 40 and also conveyed toward the laminating nip 60c. In a step prior to lamination, elastic member(s) 26 is pre-strained along the machine direction by, e.g., regulation of feed speed thereof relative to speed of laminating rollers 60a, 60 b. In another step prior to lamination, adhesive is applied to any one or any combination of layer 25a, layer 25b and elastic member(s) 26. In one example elastic member(s) 26 may be a plurality of elastic strands spaced along the cross direction, and in one example these may be coated with adhesive by a strand coating apparatus 42. In another example elastic member(s) 26 may be an elastomeric film, which also may be apertured or porous to enhance breathability of the stretch laminate. Layer 25a, elastic member(s) 26 and layer 25b, one or more of which bears applied adhesive, are then passed into lamination nip 60c where they are compressed together, causing the applied adhesive to spread among them and bond them together to form stretch laminate 25f. As noted above, it may be desired that the direction of stretch of the incremental stretching or SELFing not be parallel with the direction of stretch of the stretch laminate (here, the machine direction), and more preferably, that the direction of stretch of the incremental stretching or SELFing form an angle with the machine direction of from 45 degrees to 90 degrees.

While the foregoing description is presented within the context of features and formation of the belt structure of a balloon-style pant, it may be recognized that the manufacturing techniques described, and the resulting stretch laminate products, may have other applications. For example, the techniques and products thereof may be used to make the elastically extensible portions of components such as fastening members (also known as, *e.g.,* "ears" or "tape fasteners") of disposable tape-type diapers, waistband components, etc., including any component of a disposable absorbent article in which directional elastic stretch may be desirable.

## Claims

1. A method of forming a stretch laminate material with a highly textured appearance and feel, the stretch laminate material having a machine direction (MD) and comprising a first nonwoven layer (25a), a second nonwoven layer (25b), and an elastic member (26) disposed between the first and second nonwoven layers, comprising the steps of:
incrementally stretching or SELFing the first and second nonwoven layers;
straining the elastic member to impart an amount of pre-strain along the machine direction ;
applying adhesive to at least one of the first nonwoven, the elastic member and the second nonwoven;
passing the incrementally stretched or SELFed first and second nonwoven layers and the pre-strained elastic member along the machine direction (MD) through a nip between a pair of laminating rollers (60a, 60b) having axes of rotation along a cross direction, wherein the layers and the elastic member are compressed therebetween and bonded together by said adhesive, thereby forming the stretch laminate with the pre-strained elastic member disposed between the first and second nonwoven layers.

2. The method of claim 1 wherein the elastic member comprises a plurality of strands (26) formed of elastomeric material with their lengths in the stretch laminate substantially aligned with the machine direction (MD).

3. The method of claim 1 wherein the elastic member comprises elastic film.

4. The method of claim 2 wherein the adhesive applying step comprises coating the strands with adhesive.

5. The method of any of claims 1-4 wherein the incremental stretching step has a stretch direction (SD) and the stretch direction is not parallel with the machine direction (MD), in the stretch laminate.

6. The method of claim 5 wherein the stretch direction (SD) forms an angle of from 45 degrees to 90 degrees with the machine direction (MD).

## Patentansprüche

1. Verfahren zum Ausbilden eines Dehnungslaminatmaterials mit einem stark texturierten Erscheinungsbild und Griff, wobei das Dehnungslaminatmaterial eine Maschinenlaufrichtung (MD) aufweist und umfassend eine erste Vliesschicht (25a), eine zweite Vliesschicht (25b) und ein elastisches Element (26), das zwischen der ersten und der zweiten Vliesschicht angeordnet ist, umfassend die Schritte:
inkrementelles Dehnen oder SELF-Behandeln der ersten und der zweiten Vliesschicht;
Belasten des elastischen Elements, um ein Ausmaß von Vorbelastung entlang der Maschinenlaufrichtung zu verleihen;
Auftragen von Klebstoff auf mindestens eines von dem ersten Vlies, dem elastischen Element und dem zweiten Vlies;
Hindurchführen der inkrementell gedehnten oder SELF-behandelten ersten und zweiten Vliesschicht und des vorbelasteten elastischen Elements entlang der Maschinenlaufrichtung (MD) durch einen Walzenspalt zwischen einem Paar von Laminierwalzen (60a, 60b), die Drehachsen entlang einer Querrichtung aufweisen, wobei die Schichten und das elastische Element dazwischen komprimiert und durch den Klebstoff miteinander verbunden sind, wodurch das Dehnungslaminat mit dem vorbelasteten elastischen Element ausgebildet wird, das zwischen der ersten und der zweiten Vliesschicht angeordnet ist.

2. Verfahren nach Anspruch 1, wobei das elastische Element eine Vielzahl von Strängen (26) umfasst, die aus Elastomermaterial ausgebildet sind, wobei ihre Längen in dem Dehnungslaminat im Wesentlichen mit der Maschinenlaufrichtung (MD) ausgerichtet sind.

3. Verfahren nach Anspruch 1, wobei das elastische Element elastische Folie umfasst.

4. Verfahren nach Anspruch 2, wobei der Schritt des Auftragens von Klebstoff das Beschichten der Stränge mit Klebstoff umfasst.

5. Verfahren nach einem der Ansprüche 1 bis 4, wobei der Schritt des inkrementellen Dehnens eine Dehnungsrichtung (SD) aufweist und die Dehnungsrichtung nicht parallel zu der Maschinenlaufrichtung (MD) in dem Dehnungslaminat ist.

6. Verfahren nach Anspruch 5, wobei die Dehnungsrichtung (SD) einen Winkel von 45 Grad bis 90 Grad mit der Maschinenlaufrichtung (MD) ausbildet.

## Revendications

1. Procédé de formation d'un matériau stratifié étirable doté d'un aspect et d'un toucher fortement texturés, le matériau stratifié étirable présentant un sens machine (SM) et comprenant une première couche non tissée (25a), une seconde couche non tissée (25b), et un élément élastique (26) disposé entre les première et seconde couches non-tissées, comprenant les étapes consistant à :
étirer de manière incrémentielle ou soumettre à un étirement structurel élastique (SELFING) les première et seconde couches non tissées ;
déformer l'élément élastique pour conférer une quantité de pré-déformation le long du sens machine ;
appliquer de l'adhésif sur au moins l'un du premier non-tissé, de l'élément élastique et du second non-tissé ;
faire passer les première et seconde couches non-tissées étirées de manière incrémentielle ou soumises à un étirement structurel élastique (SELFING) et l'élément élastique précontraint le long du sens machine (SM) à travers un pincement entre une paire de galets de stratification (60a, 60b) présentant des axes de rotation le long d'une direction transversale, dans lequel les couches et l'élément élastique sont comprimés entre elles et collés ensemble par ledit adhésif, en formant ainsi le stratifié étirable avec l'élément élastique précontraint disposé entre les première et seconde couches non tissées.

2. Procédé selon la revendication 1, dans lequel l'élément élastique comprend une pluralité de brins (26) formés de matériau élastomère, leurs longueurs dans le stratifié étirable étant sensiblement alignées avec le sens machine (SM).

3. Procédé selon la revendication 1, dans lequel l'élément élastique comprend un film élastique.

4. Procédé selon la revendication 2, dans lequel l'étape d'application d'adhésif comprend le revêtement des brins avec un adhésif.

5. Procédé selon l'une quelconque des revendications 1 à 4, dans lequel l'étape d'étirement incrémentiel présente une direction d'étirement (DE) et la direction d'étirement n'est pas parallèle au sens machine (SM), dans le stratifié étirable.

6. Procédé selon la revendication 5, dans lequel la direction d'étirement (DE) forme un angle de 45 degrés à 90 degrés par rapport au sens machine (SM).
